# EUROPEAN PATENT APPLICATION

(11) **EP 2 436 370 A1**
(43) Date of publication of application: **04.04.2012**
(21) Application number: 10780597.0
(22) Date of filing: 27.05.2010
(51) Int. Cl.: A61K 8/39, A61K 8/41, A61K 8/42, A61Q 5/02, A61Q 5/12

(54) **HAIR COSMETIC**

(30) Priority: 28.05.2009 JP 2009129450
(71) Applicant: Shiseido Company, Ltd., Chuo-ku Tokyo 104-8010 (JP)
(72) Inventor: KINOSHITA Koichi, Yokohama-shi Kanagawa 224-8558 (JP)
(74) Representative: Schön, Christoph
(86) International application number: PCT/JP2010/058986
(87) International publication number: WO 2010/137643

(57) **Abstract**

The present invention provides a hair cosmetic composition contains (A) a di-long-chain cationic surfactant represented by formula (1); (B) an ethylene oxide adduct of 1,2-alkanediol represented by formula (2), and/or an N-acyl-N-methylmonoethanolamide represented by formula (3); and (C) water, wherein the number (α1) of the carbon atoms that form R^{a}CO of the di-long-chain cationic surfactant represented by formula (1) and the number (α2) of the carbon atoms that form each of R^{b}CHCH₂ of the ethylene oxide adduct of 1,2-alkanediol represented by formula (2) and R^{c}CO of the N-acyl-N-methylmonoethanolamide represented by formula (3) satisfy the relationship: (α2 - 2)≤α1≤(α2 + 2) (chemical formulas not shown). The hair cosmetic composition of the present invention has remarkably high safety and excellent stability. Also, even when the composition has a low base material content, a lamellar liquid crystal structure can be formed. Quite surprisingly, the hair cosmetic composition can provide hair with conditioning effects and shows such excellent performance as to cause no problem even though the rinsing step is omitted.

## Description

### Technical Field

The present invention relates to a hair cosmetic composition and, more particularly, to a hair cosmetic composition suitable for serving as a hair conditioner, a hair rinse, or a rinse-in-shampoo.

### Background Art

Currently, various types of hair cosmetic compositions for giving conditioning effects to hair are provided in consideration of sensation in use, ease of use, safety, etc. of users.

For example, Patent Document 1 discloses a hair cosmetic composition which contains a di-long-chain cationic surfactant, a hydrophobically modified alkylcellulose, and a higher alcohol and which forms a rinse-like gel. The hair cosmetic composition disclosed in Patent Document 1 is stable over passage of time and is suitable for serving as a hair conditioner or a hair treatment. Patent Document 2 discloses a transparent or semi-transparent conditioning composition which contains a cationic surfactant, an anionic or amphoteric polymer, and an aqueous carrier and which forms a water-insoluble complex upon dilution thereof. Patent Document 3 discloses a conditioning composition which contains a cationic surfactant, an anionic or amphoteric polymer, a high-melting-point aliphatic compound, and an aqueous carrier an which forms a gel matrix. Patent Document 4 discloses a conditioning composition which contains a cationic surfactant, an anionic or amphoteric polymer, a conditioning agent, and an aqueous carrier and which forms a water-insoluble complex between the surfactant system and the polymer.

Patent Document 5 discloses a preparation which contains a di-long-chain cationic surfactant (esterquat), an oil ingredient, and a lower alcohol, which is readily spreadable upon application, and which is rapidly absorbed without remaining residues thereof. Patent Document 6 discloses a mixture of di-long-chain cationic surfactants (esterquat) having different acyl group lengths. Patent Document 7 discloses a hair-care preparation which contains a natural oil including sterol and an unsaturated fatty acid in specific amounts.

Patent Document 8 discloses an aqueous hair cosmetic composition which contains a sucrose fatty acid ester, a cationic surfactant, a silicone, ethanol, and water and which exhibits high volatility after application, and provides hair with moistness even after volatilization of the composition.

### Prior Art Documents

### Patent Documents

Patent Document 1: Japanese Patent Application Laid-Open (*kokai*) No. 2007-112787
Patent Document 2: Japanese *Kohyo* (PCT) Patent Publication No. 2008-518490
Patent Document 3: Japanese *Kohyo* (PCT) Patent Publication No. 2008-543952
Patent Document 4: Japanese *Kohyo* (PCT) Patent Publication No. 2008-546806
Patent Document 5: Japanese *Kohyo* (PCT) Patent Publication No. 2002-527373
Patent Document 6: Japanese *Kohyo* (PCT) Patent Publication No. 2003-535108
Patent Document 7: Japanese *Kohyo* (PCT) Patent Publication No. 2004-531565
Patent Document 8: Japanese Patent Application Laid-Open (*kokai*) No. 2005-330124

### Summary of the Invention

### Problems to be Solved by the Invention

As described above, a variety of hair cosmetic compositions for giving conditioning effects to hair have been provided. However, such hair cosmetic compositions are desired to be improved for higher performance. Thus, an object of the present invention is to provide a hair cosmetic composition which provides hair with conditioning effects, which ensures safety to the scalp and skin to such a degree that the composition is not required to be rinsed off after application thereof to hair, which attains improved sensation in use after application thereof to hair, and which is stable over passage of time.

The aforementioned hair cosmetic composition disclosed in Patent Document 1 is stable over passage of time and can form a rinse-like gel. In order to enhance stability, a higher alcohol is incorporated as an essential ingredient into the composition. The invention of Patent Document 1 is directed to a technique of enhancing stability of the composition in the presence of a higher alcohol. However, the stability of products from the composition remains unsatisfactory. That is, the stability of the composition is unsatisfactory at high and low temperatures.

Some of the aforementioned compositions contain esterquat. Although the compositions as described above assumes cream to milky lotion by virtue of the presence of a higher alcohol, the other compositions are a liquid composition having very low viscosity or a composition whose viscosity has been enhanced by only a polymer thickener. Such a low-viscosity composition does not meet the users' demand, while such a composition whose viscosity has been enhanced by only a polymer thickener provides sticky sensation due to the polymer and cannot suppress irritation due to the cationic surfactant. Furthermore, there has been found no co-surfactant which is used in combination with esterquat and which provides such a composition with stability higher than that attained by a higher alcohol. Means for Solving the Problems

to [0016] The present invention has been conceived to attain the aforementioned object. Accordingly, the present invention provides a hair cosmetic composition comprising
(A) a di-long-chain cationic surfactant represented by formula (1): 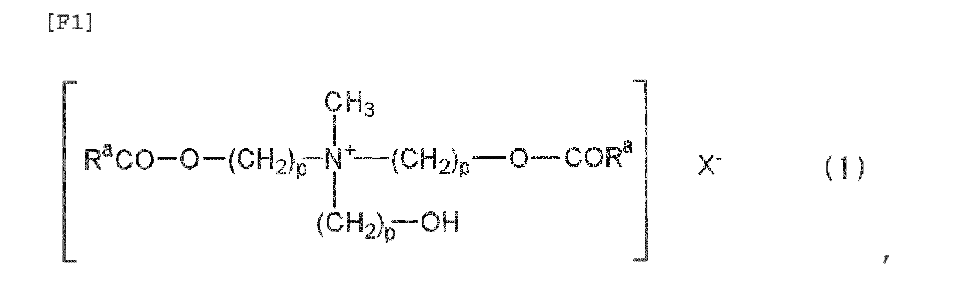 wherein each of R^{a}COs, which may be identical to or different from each other, represents an aliphatic acyl group having 10 to 22 carbon atoms and 0 to 3 double bonds; p is an integer of 1 to 3; and X represents a halogen atom, metosulfate, or metophosphate;
(B) an ethylene oxide adduct of 1,2-alkanediol represented by formula (2): 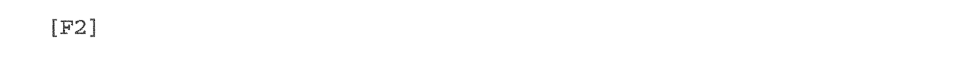 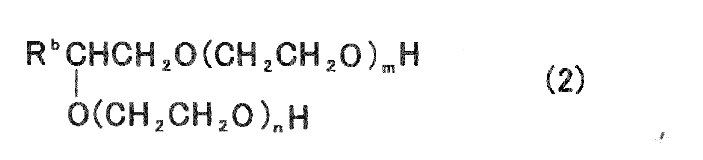 wherein R^{b} represents a hydrocarbon group having 8 to 20 carbon atoms and 0 to 2 double bonds; each of m and n, which represent amount by mol of added ethylene oxide, is an integer of 0 to 2; and the average of m+n is greater than 0 and smaller than 2; and/or an N-acyl-N-methylmonoethanolamide represented by formula (3): 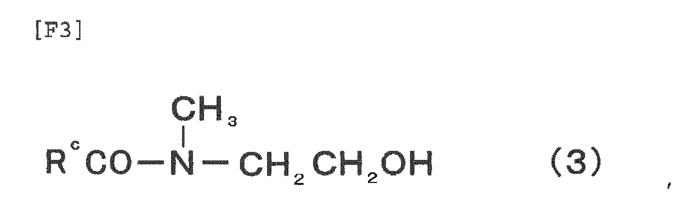 wherein R^{C} represents a hydrocarbon group having 9 to 21 carbon atoms and 0 to 2 double bonds; and
(C) water,
   wherein the number (α1) of the carbon atoms that form R^{a}CO of the di-long-chain cationic surfactant represented by formula (1) and the number (α2) of the carbon atoms that form each of R^{b}CHCH₂ of the ethylene oxide adduct of 1,2-alkanediol represented by formula (2) and R^{c}CO of the N-acyl-N-methylmonoethanolamide represented by formula (3) satisfy the relationship: (α2 - 2 )≤α1≤(α2 + 2)
(hereinafter the composition may also be referred to as the composition of the present invention).

### Effects of the Invention

The hair cosmetic composition of the present invention has remarkably high safety and excellent stability. Also, even when the composition has a low base material content, a lamellar liquid crystal structure can be formed. Quite surprisingly, the hair cosmetic composition can provide hair with conditioning effects and shows such excellent performance as to cause no problem even though the rinsing step is omitted. Specifically, the composition may be employed as a hair rinse, a hair conditioner, a rinse-in-shampoo, etc.

### Brief Description of the Drawings

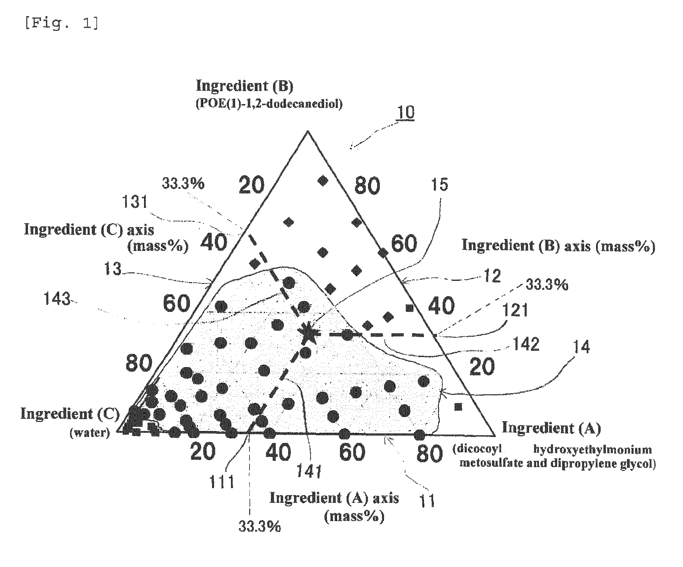

[Fig. 1] Fig. 1 represents a ternary phase diagram showing relationship of the essential ingredients of the present invention.

### Modes for Carrying Out the Invention

As described above, the hair cosmetic composition of the present invention contains, as essential ingredients, (ingredient (A)) a di-long-chain cationic surfactant (1), (ingredient (B)) an ethylene oxide adduct of 1,2-alkanediol (2), and (ingredient (C)) water.

### [Ingredient (A)]

Ingredient (A) is a di-long-chain cationic surfactant represented by formula (1).

In formula (1), R^{a}CO represents an aliphatic acyl group having 10 to 22 (preferably 12 to 16, most preferably 12) carbon atoms and 0 to 3 double bonds; p is an integer of 1 to 3; and X represents a halogen atom, metosulfate, or metophosphate.

Ingredient (A) is preferably a halide or metosulfate of a coconut fatty acid-based esterquat. Examples of the commercial product of preferred ingredient (A) include Dehyquart L80 (product of Cognis Japan) (dicocoylethyl hydroxyethylmonium metosulfate). The ingredient (A) content of the hair cosmetic composition is preferably 0.1 to 20 mass%, more preferably 0.5 to 10 mass%. When the ingredient (A) content of the hair cosmetic composition is less than 0.1 mass%, hair conditioning effects and viscosity of the composition are prone to be poor, and the long-term stability of the composition particularly at low temperature is prone to decrease. When the content is in excess of 20 mass%, hair receives excessive conditioning effects to cause stickiness, and the composition has poor safety to the scalp and skin, and insufficient viscosity and elastic property at ambient temperature.

### [Ingredient (B)]

Ingredient (B) is an ethylene oxide adduct of 1,2-alkanediol represented by formula (2) and an N-acyl-N-methylmonoethanolamide represented by formula (3). One or more embers of ingredient (B) may be incorporated into the hair cosmetic composition of the present invention.

Hereinafter, the above ethylene oxide (EO) adduct of 1,2-alkanediol may be referred to as "EO alkanediol," "EO (average of the numbers of bonded EO units) alkanediol," or "POE (average of the numbers of bonded EO units) alkanediol." As used herein, the term "average of the numbers of bonded EO units" is equivalent to the average of m+n in formula (2).

In formula (2), R^{b} represents a hydrocarbon group having 8 to 20 (preferably 10 to 14, most preferably 10) carbon atoms. In other words, R^{b}CHCH₂ has 10 to 22 (preferably 12 to 16, most preferably 12) carbon atoms. The number of added ethylene oxide units, represented by m or n, is an integer of 0 to 2. The average of m+n (the average of the sum of m and n) is greater than 0 and smaller than 2. Preferably, the average of the sum of m and n is not less than 0.5 nor more than 1.5 (the average of m+n is 0.5 to 1.5).

In formula (3), R^{c} represents a hydrocarbon group having 9 to 21 (preferably 11 to 15, most preferably 11) carbon atoms. In other words, R^{c}CO has 10 to 22 (preferably 12 to 16, most preferably 12) carbon atoms. For example, coconut fatty acid N-methylmonoethanolamide (see the Examples) has a coconut fatty acid group corresponding to R^{c}CO having 12 carbon atoms and exemplified as a preferred species of N-acyl-N-methylmonoethanolamide.

The ingredient (B) content of the hair cosmetic composition, which varies depending on the mode of use (performing rinsing or not), is preferably 0.5 to 50 mass%, more preferably 0.5 to 20 mass%. When the ingredient (B) content of the hair cosmetic composition is less than 0.5 mass%, the composition has insufficient viscosity and elastic property at ambient temperature, whereas when the content is in excess of 50 mass%, the composition is prone to have poor safety (i.e., causing irritation or allergic reaction) to the scalp and skin, and poor stability in appearance at low temperature.

### [Ingredient (C)]

As described above, ingredient (C) is water. Any water such as purified water, ion-exchange water, tap water, or natural water may be used. No particular limitation is imposed on the ingredient (C) content, but it is about 10 to about 95 mass% with respect to the entirety of the hair cosmetic composition and is adjusted such that the hair cosmetic composition of the present invention has appropriate ingredient (A) content, ingredient (B) content, and optional additive content. When the water content is excessively high, each ingredient concentration of the hair cosmetic composition becomes excessively low, failing to fully attain the effects of the present invention, whereas when the water content is excessively low, each ingredient concentration of the hair cosmetic composition becomes excessively high, resulting in mixing failure and loss of raw materials.

### [Relationship among ingredients (A) to (C)]

The amounts of essential ingredients (A) to (C) incorporated into the hair cosmetic composition of the present invention are as described above. These amounts are adjusted in consideration of some other factors.

Regarding the requirements in terms of the number of carbon atoms, the number (α1) of the carbon atoms that form R^{a}CO of the di-long-chain cationic surfactant represented by formula (1) and the number (α2) of the carbon atoms that form each of R^{b}CHCH₂ of the ethylene oxide adduct of 1,2-alkanediol represented by formula (2) and R^{c}CO of the N-acyl-N-methylmonoethanolamide represented by formula (3) preferably satisfy the relationship: (α2 - 2)≤α1≤(α2 + 2), and more preferably, α1 is equal to α2, from the viewpoint of maintaining the stability of the composition of the present invention at high level.

In an actual situation, each ingredient may be a mixture of the corresponding compounds having different numbers of carbon atoms. Thus, the concept "equal" refers not only to the case where the numbers of carbon atoms of the two species are correctly equal to each other, but also to the case where the numbers of carbon atoms of the most predominant species in the mixtures are equal to each other.

The compositional proportions of ingredients (A) to (C) preferably adjusted such that a lamellar liquid crystal structure is formed in the system. Specifically, the proportions are preferably adjusted to fall within a " lamella liquid crystal-forming area" of the ternary phase diagram (Fig. 1) Formation of the lamellar liquid crystal structure may be confirmed through an X-ray scattering method (small angle: SAXES, wide angle: WAXS), observation under a polarization microscopy, FF-TEM observation, or the like. Each ingredient content is read out from the ternary phase diagram in a manner generally known in the art.

In Fig. 1, the ternary phase diagram 10 is a diagram formed from three axes 11, 12, 13, and the triangular area defined by the axes. The axis 11 represents the amount of ingredient (A) (the total amount of dicocoylethyl hydroxyethylmonium metosulfate and propylene glycol (unit: mass% with respect to the entire of the three ingredient, and the same applies hereinafter). In the system represented by the diagram, Dehyquart L80 is employed as ingredient (A).
The ingredient (A) commercial product contains 76 mass% of ingredient (A) and 24 mass% of propylene glycol. The axis 12 represents the amount of ingredient (B) (POE(1.0)-1,2-dodecanediol, which is denoted by POE(1)-1,2-dodecanediol in Fig. 1). The axis 13 represents the amount of ingredient (C) (water).

A closed curve 14 and the shadowed area inside the closed curve 14 in the ternary phase diagram 10 represents ranges of the compositional proportions where a lamellar liquid crystal structure is formed. Each of the black circle dots in the area represents a specific system having compositional proportions at which a lamellar liquid crystal structure can be formed. Each of the diamond dots and square dots in the bright area outside the closed curve 14 represents a specific system having compositional proportions at which no lamellar liquid crystal structure is formed.

Specific values of the ingredient (A), (B), and (C) contents may be determined from the ternary phase diagram 10 through a routine method. For example, the ingredient (A) content at a star 15 in the shadowed area defined by the closed curve 14 can be derived by drawing a line 141 parallel to the axis 12 from the star 15 toward the axis 11 and reading the value at the point 111 where the parallel line and the axis 11 intersect. The value which is the ingredient (A) content is 33.3 mass%/3-ingredients. The ingredient (B including propylene glycol) content at the star 15 can be derived by drawing a line 142 parallel to the axis 13 from the star 15 toward the axis 12 and reading the value at the crossing 121 where the parallel line and the axis 12 intersect. The value is 33.3 mass%/3-ingredients. The ingredient (B) content can be calculated to be 25.3 mass% from the ratio to propylene glycol. The ingredient (C) content at the star 15 can be derived by drawing a line 143 parallel to the axis 11 from the star 15 toward the axis 13 and reading the value at the point 131 where the parallel line and the axis 13 intersect. The value which is the ingredient (C) content is 33.3 mass%/3-ingredients. Thus, it can be read out from the ternary phase diagram 10 that the star 15 represents a system in which ingredient (A), ingredient (B) and propylene glycol, and ingredient (C) are contained in an amount of 33.3 mass%/3-ingredients, respectively.

As described above, through incorporation of ingredients (A) to (C) into the hair cosmetic composition of the present invention, remarkably high safety and excellent stability can be attained. Also, even when the composition has a low base material content, a lamellar liquid crystal structure can be formed. In addition, the hair cosmetic composition can attain such excellent safety and performance as to cause no problem even though rinsing after application thereof is omitted. The hair cosmetic composition can form a base material which maintains a lamellar liquid crystal structure in a wide temperature range suitable for serving as a hair treatment or a hair conditioner. Notably, it is generally known that, through employment of a conventionally employed combination of a cationic surfactant, a higher alcohol (e.g., cetyl alcohol, stearyl alcohol, behenyl alcohol, or a mixture thereof), and water, α gel is formed during storage at room temperature or in actual application, although a lamellar liquid crystal structure is formed at high temperature (e.g., about 80°C). Thus, the present invention is quite different from such a conventional technique in terms of the structure.

### [Specific embodiments containing other ingredients]

The hair cosmetic composition of the present invention may further contain additional ingredients, so long as the effects of the present invention are not impaired. Examples of such additional ingredients include powder ingredients (e.g., mica, talc, kaolin, calcium carbonate, magnesium carbonate, silicic anhydride, aluminum oxide, barium sulfate, red iron oxide, yellow iron oxide, black iron oxide, chromium oxide, ultramarine, iron blue, carbon black, titanium dioxide, zinc oxide, titanate mica, fish scale flake, bismuth oxychloride, boron nitride, photochromic pigments, synthetic fluorphlogopite, iron-containing synthetic fluorphlogopite, and composite microparticle powder); fats and oils (liquid) (e.g., olive oil, camellia oil, macadamia nut oil, and castor oil); fats and oils (solid); waxes (e.g., carnauba wax, candelilla wax, jojoba oil, beeswax, and lanolin); hydrocarbon oils (e.g., liquid paraffin, paraffin, petrolatum, ceresin, microcrystalline wax, and squalane); higher fatty acids (e.g., lauric acid, myristic acid, palmitic acid, stearic acid, and isostearic acid); ester oils (e.g., isopropyl myristate, 2-octyldodecyl myristate, cetyl 2-ethylhexanoate, and diisostearyl malate); higher alcohols (e.g., cetyl alcohol, stearyl alcohol, and isostearyl alcohol); silicon oils (e.g., methylpolysiloxane, methylphenylpolysiloxane, methylhydrogenpolysiloxane, and silicone oils such as amino-modified or polyether-modified silicone oil); surfactants (other than ingredients (A) and (B)); humectants (e.g., glycerin, propylene glycol, dipropylene glycol, 1,3-butylene glycol, polyethylene glycol, sorbitol, sorbitol, sodium 2-pyrrolidone-5-carboxylate, and sodium hyaluronate); water-soluble polymers (e.g., cationized polysaccharides such as cationized cellulose, cationized guar gum, and cationized locust bean gum; synthetic cationic polymers such as polyquaternium-7; and amphoteric polymers such as polyquaternium-39); thickeners (e.g., plant-derived thickeners, microorganism-derived thickeners, animal-derived thickeners, cellulose-derived thickeners, starch-derived thickeners, alginic acid-derived thickeners, vinylic polymers, and high-molecular-weight polyethylene glycol); -absorbers; seauestering agents; lower alcohol; polyhydric alcohols; sugars; amino acids; organic amines; polymer emulsions; pH-regulators; skin nutrients; vitamins; anti-oxidants; antioxidant aids; various extracts; antiseptic agents (e.g., methylparaben, ethylparaben, propylparaben, phenoxyethanol, sodium benzoate, 1,2-octanediol, and methylisothiazolinone); pigments (e.g., Red No. 106, Orange No. 205, Yellow No. 4, Green No. 3, and Blue No. 1); and a perfume. Through incorporation of appropriately selected these additives into the composition of the invention, a variety of products thereof having a target form can be produced through a routine method.

The hair cosmetic composition of the present invention is suitably employed as, for example, a hair rinse, a hair conditioner, a hair treatment, or a rinse-in-shampoo. Regarding conventional hair cosmetic compositions employed in general products of hair rinse, hair treatment, rinse-in-shampoo, etc., in a typical mode of use, the composition is applied to hair, and the applied composition is rinsed off immediately after application or after maintenance with hair for a while. However, the hair cosmetic composition of the present invention does not leave unpleasant sensation to users and is free from irritation to the scalp and skin, even though the rinsing step is omitted, and hair conditioning effects can be substantially enhanced. Furthermore, in the case where the rinsing step is omitted, environmental load (CO₂ emission) due to use of hot water for rinsing is drastically reduced, and utility cost can be reduced. Examples

The present invention will next be described by way of Examples, which should not be construed as limiting the invention thereto. Unless otherwise specified, the amount of an ingredient is on the basis of mass% with respect to the entire amount of the target into which the ingredient is incorporated.

### [Details of Tests]

The test methods employed for evaluating test products in the Test Examples, and test results will be described. Table 1 shows the formulations of the tested products (Test Examples 1 to 19), and the test results. Each test product was prepared by mixing, in water, ingredients other than water at a temperature higher than that of the melting point of each ingredient, and cooling the mixture to room temperature.

### (1) Stability test

### (a) Evaluation over time

Each of the thus-prepared test products was placed in a sample bottle, and the bottle was closed with a cap. The sample bottle was allowed to stand for two weeks at a predetermined temperature. The appearance of the test product was visually observed, and the viscosity thereof was measured by means of a Brookfield viscometer. Viscosity measurement was performed after rotation of the test product at 12 rpm for one minute. The viscosity values were evaluated as follows.

### <Evaluation criteria>

(i) Storage at 30°C
   ○○: Uniform appearance, and viscosity of 1,000 mPa·s or higher
   ○: Uniform appearance, and viscosity of 100 mPa·s or higher and lower than 1,000 mPa·s
   △: Uniform appearance, and viscosity of lower than 100 mPa·s X: Non-uniform appearance

(ii) Storage at temperatures (not 30°C) <55°C and 10°C>
   *The samples rated as "X" in (i) were not tested.
   ○: The same appearance as the 30°C-storage product, and viscosity of 0.5 to 2 times that of the 30°C-storage product
   △: Appearance different from that of the 30°C-storage product, or viscosity lower than 0.5 times or higher than 2 times that of the 30°C-storage product
   X: Appearance different from that of the 30°C-storage product, and viscosity lower than 0.5 times or higher than 2 times that of the 30°C-storage product

### (2) Actual use test

Ten expert panelists were subjected to actual use tests in terms of tactile sensation in use (spreadability of the test product over hair) and smoothness of hair after drying. The method and evaluation criteria for each of the test items, are as follows.

### <Tactile sensation in use (spreadability over hair)>

After shampooing and rinsing off the shampoo, each of the samples of the Test Examples was applied to the hair of each of the ten expert panelists. The panelists evaluated "spreadability over hair" upon application of the sample of the Test Example. The Test Examples were compared on the basis of the following criteria:
○: Eight or more panelists responded "good spreadability"
△: Five to seven panelists responded "good spreadability"
X: Four or less panelists responded "good spreadability"
-: Test is not performed because the appearance of the test product is not uniform.

### <Hair smoothness after drying>

Each of the samples of the Test Examples was applied to the hair of each of the ten expert panelists, after shampooing and rinsing off. The panelists evaluated "hair smoothness" after drying hair without rinsing off the applied product. The Test Examples were compared on the basis of the following criteria:
○: Eight or more panelists responded "smooth"
△: Five to seven panelists responded "sooth"
X: Four or less panelists responded "smooth"
-: Washing is required due to possible irritation to the scalp and skin in view of the amount of any ingredient, or, test is not performed because the appearance of the test product is not uniform.

[Table 1]

**[Table 1-1]**

| | Test Ex. 1 | Test Ex. 2 | Test Ex. 3 | Test Ex. 4 | Test Ex. 5 | Test Ex. 6 | Test Ex. 7 |
|---|---|---|---|---|---|---|---|
| Materials | Amount (mass%) | | | | | | |
| Dehyquart L80 | 3.0 | 3.0 | 3.0 | 3.0 | 3.0 | 3.0 | 3.0 |
| Dehyquart AU56/G | - | - | - | - | - | - | - |
| Stearyl trimethyl ammonium chloride | - | - | - | - | - | - | - |
| Sodium methyl stearoyl taurate | - | - | - | - | - | - | - |
| 1,2-dodecanediol | 9.0 | - | - | - | - | - | - |
| POE(0,5)-1,2-dodecanediol | - | 8.0 | - | - | - | - | |
| POE(0.8)-1,2-dodecanediol | - | - | 9.0 | - | - | - | - |
| POE(1)-1,2-dodecanediol | - | - | - | 9.0 | - | - | - |
| POE(1.5)-1,2-dodecanediol | - | - | - | - | 9.0 | - | - |
| POE(2)-1,2-dodecanediol | - | - | - | - | - | 9.0 | - |
| Coconut fatty acid N-methylmonoethanolamide | - | - | - | - | - | - | 9.0 |
| Cetostearyl alcohol | - | - | - | - | - | - | - |
| POE(1)-cetyl alcohol | - | - | - | - | - | - | - |
| Propylene glycol laurate | - | - | - | - | - | - | - |
| Glyceryl monooleate | - | - | - | - | - | - | - |
| Cetyl palmitate | - | - | - | - | - | - | - |
| Coconut fatty acid diethanolamide | - | - | - | - | - | - | - |
| Cocamidopropyl dimethylamine | - | - | - | | - | - | - |
| Adekanol GT-700 | - | - | - | - | - | - | |
| Sangelose 90L | - | - | - | - | - | - | |
| Water | bal. | bal. | bal. | bal. | bal. | bal. | bal. |
| Results | | | | | | | |
| Appearance (30°C) | X | ○ | ○ | ○ | ○ | X | ○ |
| Appearance (55°C) | - | ○ | ○ | ○ | ○ | - | ○ |
| Appearance (10°C) | - | X | ○ | ○ | ○ | - | ○ |
| Spreadability | - | ○ | ○ | ○ | ○ | - | ○ |
| Smoothness after drying | - | ○ | ○ | ○ | ○ | - | ○ |

**[Table 1-2]**

| | Test Ex. 8 | Test Ex. 9 | Test Ex. 10 | Test Ex. 11 | Test Ex. 12 | Test Ex. 13 |
|---|---|---|---|---|---|---|
| Materials | Amount (mass%) | | | | | |
| Dehyquart L80 | 2.0 | 2.0 | 2.0 | 2.0 | 3.0 | 3.0 |
| DehyquartAU56/G | - | - | - | - | - | - |
| Stearyl trimethyl ammonium chloride | - | - | - | - | - | - |
| Sodium methyl stearoyl taurate | - | - | - | - | - | - |
| 1,2-dodecanediol | - | - | - | - | - | - |
| POE(0.5)-1,2-dodecanediol | - | - | - | - | - | - |
| POE(0.8)-1,2-dodecanediol | - | - | - | - | - | - |
| POE(1)-1,2-dodecanediol | - | - | - | - | - | - |
| POE(1.5)-1,2-dodecanediol | - | - | - | - | - | - |
| POE(2)-1,2-dodecanediol | - | - | - | - | - | - |
| Coconut fatty acid N-methylmonoethanolamide | - | - | - | - | - | - |
| Cetostearyl alcohol | 5.0 | 5.0 | 5.0 | - | - | - |
| POE(1)-cetyl alcohol | - | - | - | 5.0 | - | - |
| Propylene glycol laurate | - | - | - | - | 9.0 | - |
| Glyceryl monooleate | - | - | - | - | - | 9.0 |
| Cetyl palmitate | - | - | - | - | - | |
| Coconut fatty acid diethanolamide | - | - | - | - | - | - |
| Cocamidopropyl dimethylamine | - | - | - | - | - | - |
| Adekanol GT-700 | - | 0.4 | - | - | - | - |
| Sangelose 90L | - | - | 0.2 | - | - | - |
| Water | bal. | bal. | bal. | bal. | bal. | bal. |

| Results | | | | | | |
|---|---|---|---|---|---|---|
| Appearance (30°C) | ○ | ○ | ○ | ○ | X | X |
| Appearance (55°C) | X | X | X | X | - | - |
| Appearance (10°C) | - | - | - | - | - | - |
| Spreadability | △ | △ | △ | △ | - | - |
| Smoothness after drying | ○ | △ | △ | ○ | - | - |

**[Table 1-3]**

| | Test Ex. 14 | Test Ex. 15 | Test Ex. 16 | Test Ex. 17 | Test Ex. 18 | Test Ex. 19 |
|---|---|---|---|---|---|---|
| Materials | Amount (mass%) | | | | | |
| Dehyquart L80 | 2.0 | 3.0 | 2.0 | - | - | - |
| Dehyquart AU56/G | - | - | - | 3.0 | - | - |
| Stearyl trimethyl ammonium chloride | - | - | - | - | 2.0 | - |
| Sodium methyl stearoyl taurate | - | - | - | - | - | 2.0 |
| 1,2-dodecanediol - | - | - | - | - | - | - |
| POE(0.5)-1,2-dodecanediol | - | - | - | - | - | - |
| POE(0.8)-1,2-dodecanediol | - | - | - | - | - | - |
| POE(1)-1,2-dodecanediol | - | - | - | 6.0 | 6.0 | 6.0 |
| POE(1.5)-1,2-dodecanediol | - | - | - | - | - | - |
| POE(2)-1,2-dodecanediol | - | - | - | - | - | - |
| Coconut fatty acid N-methylmonoethanolamide | - | - | - | - | - | - |
| Cetostearyl alcohol | - | - | - | - | - | - |
| POE(1)-cetyl alcohol | - | - | - | - | - | - |
| Propylene glycol laurate | - | - | - | - | - | - |
| Glyceryl monooleate | - | - | - | - | - | - |
| Cetyl palmitate | 5.0 | - | - | - | - | - |
| Coconut fatty acid diethanolamide | - | 9.0 | - | - | - | - |
| Cocamidopropyl dimethylamine | - | - | 6.0 | - | - | - |
| Adekanol GT-700 | - | - | - | - | - | - |
| Sangelose 90L | - | - | - | - | - | - |
| Water | bal. | bal. | bal. | bal. | bal. | bal. |

| Results | | | | | | |
|---|---|---|---|---|---|---|
| Apearance (30°C) | X | X | △ | X | ○ | ○ |
| Apearance (55°C) | - | - | X | - | ○ | ○ |
| Apearance(10°C) | - | - | - | - | X | X |
| Spreadability | - | - | - | - | ○ | △ |
| Smoothness after drying | - | - | - | - | △ | X |

In Table 1, the employed commercial products are as follows:
Dehyquart L80: product of Cognis Japan (76% dicocoylethyl hydroxyethylmonium metosulfate, 24% propylene glycol) Dehyquart AU56/G: product of Cognis Japan (91% dipalmitoylethyl hydroxyethylmonium metosulfate)
Adekanol GT-700: product of Asahi Denka (100% PEG-240/HDI copolymer bis-decyltetradeceth-20 ether)
Sangelose-90L: product of Daido Chemical Corporation (100% hydroxypropylmethylcellulose stearcaxy ether)

As is clear from table 1, Test Example products (2, 3, 4, 5, and 7) falling within the scope of the hair cosmetic composition of the present invention were satisfactory in the stability test and actual use test. Among these products, the product of Test Example 2, in which the number of added EO of EO-dodecanediol was the smallest (lower limit), exhibited slightly poor stability at low temperature, but the other properties were good.

In the products of Test Examples falling outside the scope of the hair cosmetic composition of the present invention, non-allowable problems occurred. The Test Examples were as follows:
Test Example 1 (removal of EO-dodecanediol);
Test Example 6 (the number of added EO of EO-dodecanediol is excessive);
Test Examples 8 to 10 (use of cetostearyl alcohol instead of EO-dodecanediol);
Test Example 11 (use of EO-cetyl alcohol instead of EO-dodecanediol);
Test Example 12 (use of propylene glycol laurate instead of EO-dodecanediol);
Test Example 13 (use of glyceryl monooleate instead of EO-dodecanediol);
Test Example 14 (use of cetyl palmitate instead of EO-dodecanediol);
Test Example 15 (use of coconut fatty acid diethanolamide instead of coconut fatty acid N-methylethanolamide);
Test Example 16 (use of cocamidopropyl dimethylamine instead of coconut fatty acid N-methylethanolamide);
Test Example 17 (use of dipalmitoylethyl hydroxyethylmonium metosulfate instead of dicocoylethyl hydroxyethylmonium metosulfate, difference between α1 and α2 in Test Example 17 being 4);
Test Example 18 (use of (A) mono-long-chain cationic surfactant as ingredient (A), formulation exhibiting poor stability at low temperature and slightly poor hair smoothness after drying); and
Test Example 19 (use of sodium methyl stearoyl taurate instead of dicocoylethyl hydroxyethylmonium metosulfate).

Hereinafter, Formulation Examples of the hair cosmetic compositions of the present invention will be disclosed Commercial products used in the Formulation Example are the same as described in relation to Table 1. Equivalent names listed in "Japanese Cosmetic Labelin Name" are also given. The formulations may be prepared through the method of the Test Examples or a method generally known in the art.

### [Formulation Example 1] Hair treatment

| Formulation | Amount (mass%) |
|---|---|
| Dehyquart L80 | 7.0 |
| POE(0.9)-1,2-dodecanediol | 20.0 |
| Dimethicone (50cs) | 3.0 |
| Dimethicone (1,000,000 cs) | 0.5 |
| Aminopropylmethicone | 0.5 |
| Octyl palmitate | 0.1 |
| Methylparaben | 0.1 |
| Diglycerin | 10.0 |
| Propylene glycol | 0.5 |
| Hydroxyethylurea | 0.1 |
| PEG-90M | 0.1 |
| Hydroxyethylcellulose | 0.2 |
| Phenoxyethanol | 0.4 |
| Perfume | 0.2 |
| Ion-exchange water | bal. |
| Total | 100.0 |

### [Formulation Example 2] Hair conditioner

| Formulation | Amount (mass%) |
|---|---|
| Dehyquart L80 | 4.0 |
| POE(1.2)-1,2-dodecanediol | 15.0 |
| Dimethicone (50cs) | 3.0 |
| Amodimethicone | 0.5 |
| Bis-isobutyl PEG-14/amodimethicone copolymer | 0.05 |
| PCA dimethicone | 0.1 |
| Mineral oil | 0.1 |
| Glycerol monooleate | 0.2 |
| Octoxyglycerin | 0.1 |
| Sorbitol | 5.0 |
| 1,3-Butylene glycol | 0.5 |
| Cationized locust bean gum | 0.2 |
| Adekanol GT-700 | 0.2 |
| Benzyl alcohol | 0.1 |
| Sodium salicylate | 0.1 |
| Rose extract | 0.1 |
| Taurine | 0.1 |
| L-Arginine hydrochloride | 0.1 |
| Perfume | 0.2 |
| Ion-exchange water | bal. |
| Total | 100.0 |

### [Formulation Example 3] Rinse-in-shampoo

| Formulation | Amount (mass%) |
|---|---|
| Dehyquart L80 | 8.0 |
| POE(0.9)-1,2-dodecanediol | 30.0 |
| Cationized cellulose | 0.3 |
| Cationized guar gum | 0.2 |
| Polyquaternium 7 | 0.1 |
| Aminopropylmethicone | 0.2 |
| Dimethicone (50cs) | 0.5 |
| Methylparaben | 0.1 |
| Propylparaben | 0.1 |
| Diglycerin | 5.0 |
| 1,3-Butylene glycol | 0.5 |
| Citric acid | 0.05 |
| Na citrate | 0.05 |
| Hydrolyzed wheat protein | 0.05 |
| Soybean extract | 0.2 |
| Perfume | 0.2 |
| Ion-exchange water | bal. |
| Total | 100.0 |

## Claims

1. A hair cosmetic composition comprising
(A) a di-long-chain cationic surfactant represented by formula (1): 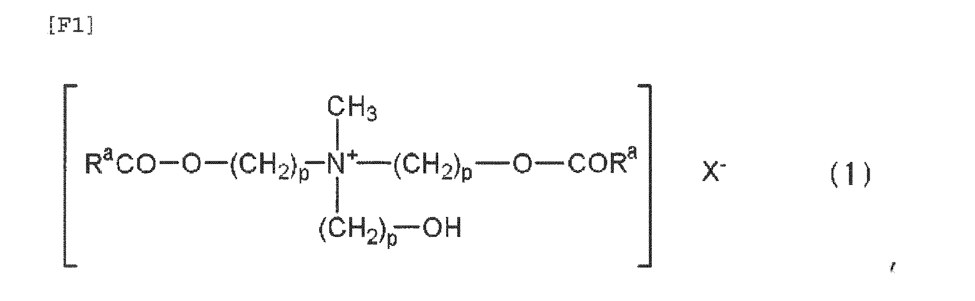 wherein each of R^{a}COs, which may be identical to or different from each other, represents an aliphatic acyl group having 10 to 22 carbon atoms and 0 to 3 double bonds; p is an integer of 1 to 3; and X represents a halogen atom, metosulfate, or metophosphate;
(B) an ethylene oxide adduct of 1,2-alkanediol represented by formula (2): 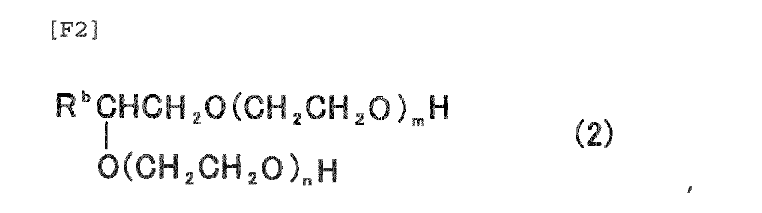 wherein R^{b} represents a hydrocarbon group having 8 to 20 carbon atoms and 0 to 2 double bonds; each of m and n, which represent amount by mol of added ethylene oxide, is an integer of 0 to 2; and the average of m+n is greater than 0 and smaller than 2; and/or an N-acyl-N-methylmonoethanolamide represented by formula (3): 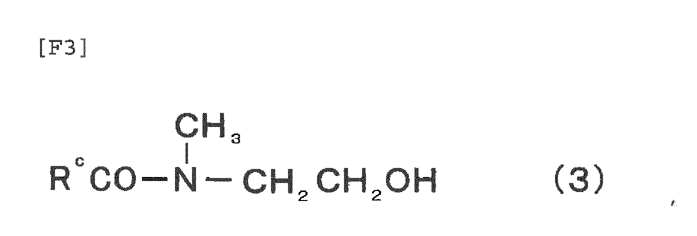 wherein R^{C} represents a hydrocarbon group having 9 to 21 carbon atoms and 0 to 2 double bonds; and
(C) water,
wherein the number (α1) of the carbon atoms that form R^{a}CO of the di-long-chain cationic surfactant represented by formula (1) and the number (α2) of the carbon atoms that form each of R^{b}CHCH₂ of the ethylene oxide adduct of 1,2-alkanediol represented by formula (2) and R^{c}CO of the N-acyl-N-methylmonoethanolamide represented by formula (3) satisfy the relationship: (α2- 2)≤α1≤(α2 + 2).

2. The hair cosmetic composition according to claims 1, wherein the R^{a}CO of the di-long-chain cationic surfactant (1) has 10 to 14 carbon atoms.

3. The hair cosmetic composition according to claim 1 or 2, wherein each of R^{b}CHCH₂ of the ethylene oxide adduct of 1,2-alkanediol (2) and R^{c}CO of N-acyl-N-methylmonoethanolamide (3) has 10 to 14 carbon atoms.

4. The hair cosmetic composition according to any of claims 1 to 3, wherein the average of the sum of m and n is not less than 0.5 nor more than 1.5, each of m and n representing amount by mol of added ethylene oxide.

5. The hair cosmetic composition according to any of claims 1 to 4, wherein the di-long-chain cationic surfactant (1) is dicocoylethyl hydroxyethylmonium metosulfate.

6. The hair cosmetic composition according to any of claims 1 to 5, which has a lamellar liquid crystal structure at 30°C.

7. The hair cosmetic composition according to any of claims 1 to 6, which is a hair conditioner, a hair rinse, or a rinse-in-shampoo.

8. The hair cosmetic composition according to any of claims 1 to 7, which is used without rinsing off the composition after application thereof.
